# EUROPÄISCHE PATENTSCHRIFT

(11) **EP 1 149 841 B1**
(45) Veröffentlichungstag und Bekanntmachung des Hinweises auf die Patenterteilung: **28.10.2009**
(21) Anmeldenummer: 01250278.7
(22) Anmeldetag: 11.08.1997
(51) Int. Cl.: C07J 53/00

(54) **Verfahren zur Herstellung von 6B,7B;15B,16B-dimethylene-5B-hydroxy-3-oxo-17alpha-androstane-21,17-carbolactone**
Process for the production of 6B,7B;15B,16B-dimethylene-5B-hydroxy-3-oxo-17alpha-androstane-21,17-carbolactone
Procédé de production de 6B,7B;15B,16B-diméthylene-5B-hydroxy-3-oxo-17alpha-androstane-21,17-carbolactone

(30) Priorität: 12.08.1996 DE 19633685
(43) Veröffentlichungstag der Anmeldung: 31.10.2001
(62) Teilanmeldung aus: 97937562.3
(73) Patentinhaber: Bayer Schering Pharma Aktiengesellschaft, 13353 Berlin (DE)
(72) Erfinder: Mohr, Jörg-Thorsten, 59423 Unna (DE); Nickisch, Klaus, 12307 Berlin (DE)

(56) Entgegenhaltungen:
- EP-A- 0 051 143
- EP-A- 0 075 189
- WO-A-90/14344
- D. BITTLER ET AL: "Synthesis of Spirorenone - A Novel Highly Active Aldosterone Antagonist" ANGEWANDTE CHEMIE. INTERNATIONAL EDITION., Bd. 21, Nr. 9, 1982, Seiten 696-697, XP002047531 WEINHEIM DE

## Beschreibung

Die Erfindung betrifft Verfahren zur Herstellung von 6β,7β;15β,16β-Dimethylene-5β-hydroxy-3-oxo-17α-androstane-21,17-carbolactone (5β-OH-DRSP), einem Vorprodukt von Drospirenon.

Drospirenon (6β,7β;15β,16β-dimethylene-3-oxo-17α-pregn-4-ene-21,17-carbolactone, **DRSP,** INN) ist als steroidaler Wirkstoff seit längerem bekannt (DE 26 52 761 C2 und DE 30 22 337 A1) und die Herstellung der letzten 4 Schritte erfolgt im Eintopfverfahren; bei dem nach der Hydrierung von Dimethylenpropinol keine der durchlaufenen Zwischenstufen Dimethylenpropanol und 5-β-OH-DRSP isoliert werden (siehe nachfolgendes Schema).

Eine analoge Synthese, jedoch unter Anwendung eine Pyridiniumdichchromat - Oxidation ist aus dem Stand der Technik bekannt [Angew. Chemie, 21, 9, (1982), Seiten 696-697)]. Ähnliche Synthesen zur Herstellung von steroidalen 17, 21-Carbolactonen werden auch innerhalb von EP-A-0 075 189 und EP-A-0 051 143 beschrieben, jedoch unter Beteiligung von mikrobiologischen Reaktionen. Oxidationen unter Beteiligung von Rutheniumverbindungen werden aber nicht darin offenbart.

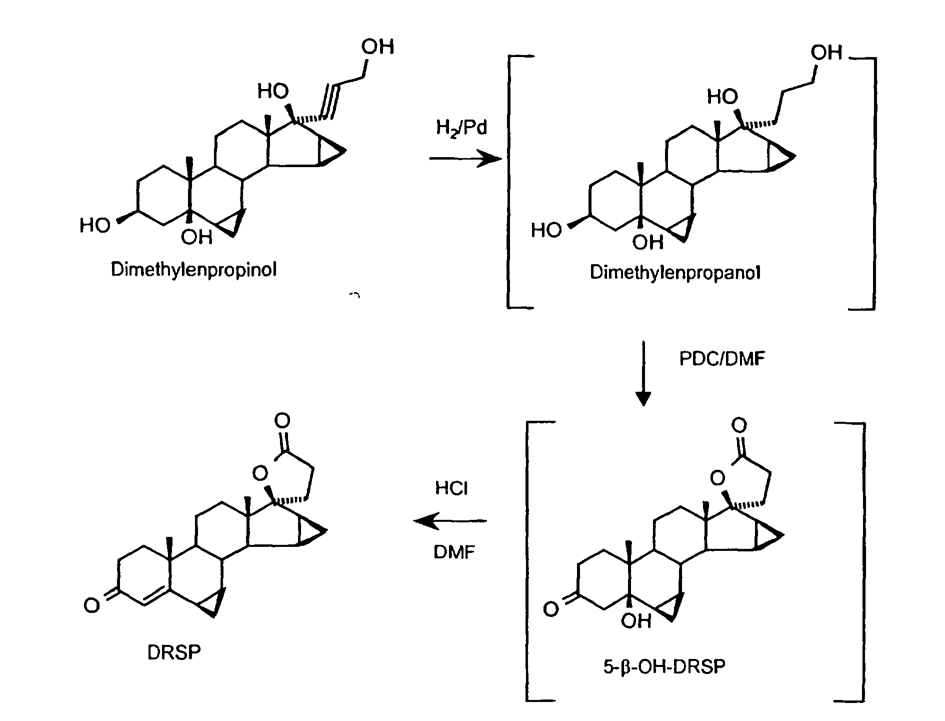

Das Dimethylenpropinol wird in THF mit Wasserstoff an Palladium-Kohle zum Dimethylenpropanol hydriert. Die so erhaltene Hydrierlösung, die das Propanol als Hauptprodukt und schwankende Anteile an Lactol enthält, wird ohne Isolierung und Zwischenaufarbeitung zum Drospirenon (DRSP) umgesetzt.

Hierzu wird zuerst ein Lösungsmittelwechsel von THF zu DMF vollzogen und anschließend das Propanol bei 40° C mit einem Überschuß von 3,7 Equivalenten Pyridiniumdichromat (PDC) zu einem Gemisch von DRSP und 5-β-OH-DRSP oxidiert. Die 5-β-OH-Funktion im Oxidationsprodukt ist labil gegenüber Säuren, Lewissäuren und basischen Bedingungen bei erhöhten Temperaturen, da in allen Fällen mit der Ausbildung des Δ-4,5-ungesättigten Ketons im Drospirenon ein thermodynamisch stabileres Produkt erhalten wird. Die Eliminierung der β-OH-Funktion im 5-β-OH-DRSP verläuft zum thermodynamisch stabileren Drospirenon und konnte nicht unterdrückt werden konnte.

Die Mischung enthält in der Regel wechselnde Anteile der beiden Komponenten, wobei das 5-β-OH-DRSP im allgemeinen als Hauptkomponente im Verhältnis von 2-3:1 vorliegt. In der letzten Stufe der Eintopfsequenz wird das Zweikomponenten-Gemisch durch Zugabe von halbkonzentrierter Salzsäure in das DRSP, roh überführt.

In der nachstehenden Tabelle sind die letzten vier Betriebsansätze zusammengefaßt.

| Ansatz | Ausbeute, roh (%) | Reinheit (100%-Methode) |
|---|---|---|
| 1 | 57,2 | 98,9 |
| 2 | 63,7 | 99,09 |
| 3 | 46,5 | 99,18 |
| 4 | 58,3 | 98,81 |
| **Gesamt** | **mittlere Ausbeute: 56,4** | **mittlere Reinheit: 98,9** |

Im Mittel aller Betriebsansätze wird ausgehend vom Dimethylenpropinol eine theoretische Ausbeute von 56% DRSP, roh in einer HPLC-Reinheit von 98,9% erzielt.

Aufgabe der Erfindung ist die Bereitstellung eines neuen Herstellungsverfahrens für Drospirenon, welches selektiver und einfacher in der Durchführung ist, als jenes aus dem Stand der Technik und außerdem ökologischer ist; (Einsparung einer Chromtrioxid-Oxidation).

Gelöst wird diese Aufgabe gemäß der Lehre des Anspruchs.

Die vorliegende Erfindung beinhaltet ein Verfahren zur Herstellung des Vorproduktes von Drospirenon
(6β,7β; 15β,16β-dimethylene-5-β-hydroxy-3-oxo-17α-androstan-21,17-carbolactone). 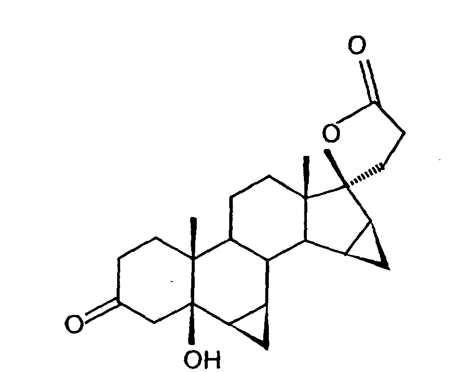 Das Ausgangsprodukt des beanspruchten Verfahrens wird durch katalytische Hydrierung von 17α-(3-hydroxy-1-propynyl)-6β,7β;15β,16β-dimethylene-5β-androstane-3β,5,17β-triol hergestellt: 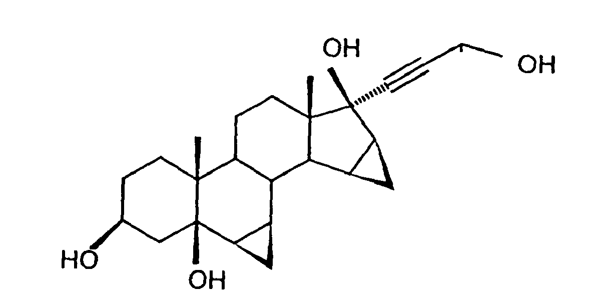 Das Ausgangsprodukt, 7α-(3-hydroxy-1-propyl)-6β,7β; 15β,16β-dimethylene-5β-androstane-3β,5,17β-triol ,

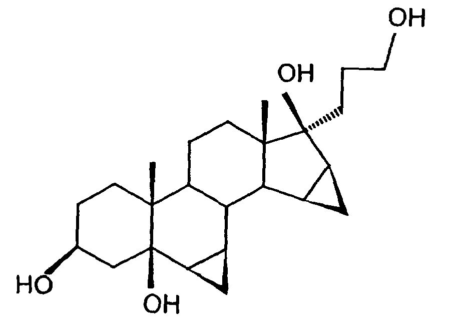

wird erfindungsgemäß durch Oxidation in Gegenwart katalytischer Mengen eines Rutheniumsalzes in das 6β,7β;15β,16β-dimethylene-5 ß-hydroxy-3-oxo-17α-androstane-21,17-carbolactone umgesetzt.

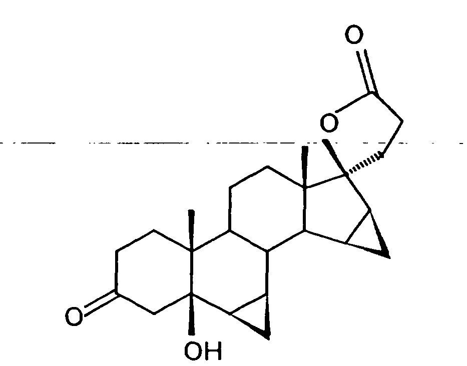

Die vorliegende Erfindung besteht aus der Schlüsselreaktion der Ruthenium katalysierte Oxidation von Dimethylenpropanol zum 5-β-OH-DRSP.

Analog zum bekannten Verfahren aus dem Stand der Technik wird Dimethylenpropinol in THF mit Wasserstoff an Palladium-Kohle hydriert. Die Hydrierlösung wird anschließend einem Lösungsmittelwechsel von THF auf Acetonitril unterworfen. Die Acetonitril-Lösung wird mit einer katalytischen Menge Rutheniumtrichlorid (1mol%) und 3 Equivalenten Natriumbromat bei 40°-60° C gezielt zum 5-β-OH-DRSP oxidiert.
Trotz der großen Labilität des 5-β-OH-DRSP gegenüber Säuren, Lewissäuren wie beispielsweise der Chromverbindungen im alten Betriebsverfahren, starken Basen oder hohen Temperaturen, die in allen Fällen auf die hohe Triebkraft zur Bildung des thermodynamisch stabileren Δ-4,5-ungesättigten Ketons zurückzuführen ist, gelingt unter den gewählten Reaktionsbedingungen die selektive Synthese des 5-β-OH-DRSP, ohne das eine Drospirenonbildung zu beobachten ist. Das 5-β-OH-DRSP kann durch eine (betrieblich) einfach durchzuführende Wasserfällung aus der Reaktionslösung isoliert werden.

Die Ausbeuten liegen im Bereich von 68% bis 75% über die beiden Stufen Hydrieren und anschließende Oxidation.

Ein weiterer sehr wesentlicher Vorteil des erfindungsgemäßen Verfahrens gegenüber dem Stand der Technik liegt im Bereich der Ökologie. Es ist gelungen, die bisher verwendeten toxischen Chromverbindungen, die in Form der PDC-Salze bislang zur Oxidation verwendet wurden und hinterher in Form ihrer Lösungen entsorgt werden müssen, durch katalytische Mengen eines Metalls zu ersetzen. Zudem ist es möglich, daß eingesetzte Acetonitril-Wasser-Gemisch durch azeotrope Destillation zu recyclen, so daß auch keine Gefahr für die Umwelt zu erwarten ist.

### Beispiel

### 6β,7β;15β,16β-dimethylene-5β-hydroxy-3-oxo-17α-androstane-21,17-carbolactone

50g 17α-(3-Hydroxy-1-propynyl)-6β,7β;15β,16β-dimethylene-5β-androstane-3β,5,17 β-triol wird in 1000 ml THF in der Gegenwart von 10 g Palladium auf Kohle (10%) und 3 ml Pyridin hydriert bis 2 Equivalente Wasserstoff aufgenommen werden. Anschließend wird der Katalysator abfiltriert und die Lösung bis zur Trockne eingeengt, wobei 52,7 g 7α-(3-hydroxy-1-propyl)-6β,7β; 15β,16β-dimethylene-5β-androstane-3β ,5,17β-triol ergalten werden, die ohne Reinigung weiter umgesetzt werden.

50,2g 7α-(3-hydroxy-1-propyl)-6β,7β;15β,16β-dimethylene-5β-androstane-3β,5,17β-triol werden in 250 ml Acetonitril suspendiert und auf 45° C erwärmt. Hierzu werden 0,52g Rutheniumtrichlorid gelöst in 10 ml Wasser und 62,46 g Natriumbromat gelöst in 250 ml Wasser getropft. Es wird 2 Stunden bei 50° C nachgerührt und die Lösung anschließend durch Zugabe von 1000 ml Wasser gequencht. Es werden 200 ml Ethylacetat zugesetzt, die Phasen getrennt und anschließend die wässrige Phase mit 600 ml Ethylacetat extrahiert. Die vereinigten organischen Phasen werden über Natriumsulfat getrocknet und anschließend bis zur Trockne eingeengt. Hierbei werden 43,44g 6β,7β;15β,16β-Dimethylene-5β-hydroxy-3-oxo-17α-androstane-21,17-carboactone als Rohprodukt erhalten. Durch Umkristallisieren aus Aceton-lsoether erhält man 35,7g 6β,7β;15β,16β-Dimethylene-5β-hydroxy-3-oxo-17α-androstane-21,17-carbolactone mit einem Schmelzpunkt von 216°-218°C. Der Drehwert liegt bei -65,6° (Natriumlinie, c=1,02 in CHCI3).

## Patentansprüche

1. Verfahren zur Herstellung von 6β,7β;16β,16β-Dimethylene-5β-hydroxy-3-oxo-17α-androstane-21,17-carbolactone (**5-**β**-OH-DRSP**) durch Oxidation von 17α-(3-hydroxy-1-propyl)-6β,7β;15β,16β-dimethylene-5β-androstane-3β,5,17β-triol in Gegenwart katalytischer Mengen eines Rutheniumsalzes.

## Claims

1. Process for preparing 6β,7β;15β,16β-dimethylene-5β-hydroxy-3-oxo-17α-androstane-21,17-carbolactone, (**5-**β**-OH-DRSP**) by oxidation of 17α-(3-hydroxy-1-propyl)-6β,7β;15β,16β-dimethylene-5β-androstane-3β,5,17β-triol in the presence of catalytic amounts of a ruthenium salt.

## Revendications

1. Procédé de production de 6β,7β;15β,16β-diméthylène-5β-hydroxy-3-oxo-17α-androstane-21,17-carbolactone (**5-**β**-OH-DRSP**) par oxydation de 17α-(3-hydroxy-1-propyl)-6β,7β;15β,16β-diméthylène-5β-androstane-3β,5,17β-triol en présence de quantités catalytiques d'un sel de ruthénium.
